# EUROPEAN PATENT APPLICATION

(11) **EP 4 620 943 A1**
(43) Date of publication of application: **24.09.2025**
(21) Application number: 23891579.7
(22) Date of filing: 14.11.2023
(51) Int. Cl.: C07C 67/38, B01J 23/44, C07B 61/00, C07C 69/753

(54) **METHOD FOR PRODUCING COMPOUND HAVING DICARBOXYLIC ACID DERIVATIVE STRUCTURE**

(30) Priority: 15.11.2022 JP 2022182584
(71) Applicant: Kyushu University, National University Corporation, Nishi-ku Fukuoka-shi Fukuoka 819-0395 (JP); Resonac Corporation, Tokyo 105-7325 (JP)
(72) Inventor: ISHIBASHI, Yoshitaka, Tokyo 105-7325 (JP); SHIM, Jooyoung, Fukuoka-shi, Fukuoka 819-0395 (JP); TOKUNAGA, Makoto, Fukuoka-shi, Fukuoka 819-0395 (JP); MURAYAMA, Haruno, Fukuoka-shi, Fukuoka 819-0395 (JP); YAMAMOTO, Eiji, Fukuoka-shi, Fukuoka 819-0395 (JP); SHIRAKURA, Nao, Fukuoka-shi, Fukuoka 819-0395 (JP)
(74) Representative: Strehl & Partner mbB
(86) International application number: PCT/JP2023/040934
(87) International publication number: WO 2024/106432

(57) **Abstract**

A method for producing a compound having a dicarboxylic acid derivative structure includes a reaction step of reacting an olefin, an alcohol, carbon monoxide, and oxygen with each other in the presence of a heterogeneous catalyst in which a carrier supports a palladium-containing compound, and an ammonium salt. The ammonium salt is preferably ammonium chloride. The olefin is preferably an alicyclic olefin.

## Description

### TECHNICAL FIELD

The present invention relates to a method for producing a compound having a dicarboxylic acid derivative structure.

Priority is claimed on Japanese Patent Application No. 2022-182584, filed on November 15, 2022, the content of which is incorporated herein by reference.

### BACKGROUND ART

In the related art, a compound having a dicarboxylic acid derivative structure, which is obtained by introducing two carboxy groups and/or carboxylic acid ester groups in total into a carbon-carbon double bond of an olefin (unsaturated hydrocarbon), has been used as a raw material of various compounds, an additive to a resin, and the like.

As a method for producing the compound having such a dicarboxylic acid derivative structure, a method disclosed in Patent Document 1 or Patent Document 2 can be used.

Patent Document 1 discloses a method for producing tricyclo[5.2.1.0^{2,6}]dec-3-ene-8.9-dicarboxylic acid diester, in which dicyclopentadiene, carbon monoxide, an alcohol and/or an acetal, a ketal, or an alkyl orthoformate, which is a derivative of the alcohol, are reacted with each other in the presence of a palladium catalyst, a copper or iron compound, and/or oxygen.

Patent Document 2 discloses a method for producing norbornenes by reacting norbornenes with an alcohol, carbon monoxide, and oxygen in the presence of a catalyst containing a palladium metal or a compound thereof, a copper compound, a chlorine compound, and a manganese or zinc compound, in which two carboxylic acid ester groups are introduced into a carbon-carbon double bond of the norbornenes.

### Citation List

### Patent Documents

[Patent Document 1] Japanese Unexamined Patent Application, First Publication No. S60-104039 (A)
[Patent Document 2] Japanese Unexamined Patent Application, First Publication No. H7-138205 (A)

### SUMMARY OF INVENTION

### Technical Problem

However, in a conventional method of producing the compound having the dicarboxylic acid derivative structure by reacting an olefin with an alcohol, carbon monoxide, and oxygen, and thus introducing two carboxy groups and/or carboxylic acid ester groups in total into a carbon-carbon double bond of the olefin, it has been required to improve the yield of the compound having the dicarboxylic acid derivative structure.

The present invention has been made in view of the above circumstances, and an object of the present invention is to provide a production method capable of producing a compound having a dicarboxylic acid derivative structure with a high yield, by introducing two carboxy groups and/or carboxylic acid ester groups in total into a carbon-carbon double bond of an olefin.

The "dicarboxylic acid derivative structure" in the present invention means a structure having two or more of at least one of a carboxy group or a carboxylic acid ester group in total.

### Solution to Problem

In order to solve the above-described problems, the present inventors have conducted intensive studies focusing on a catalyst used for producing the compound having the dicarboxylic acid derivative structure by reacting an olefin with an alcohol, carbon monoxide, and oxygen, and thus introducing two carboxy groups and/or carboxylic acid ester groups in total into a carbon-carbon double bond of the olefin.

As a result, it is found that, as the catalyst, it is sufficient to use a heterogeneous catalyst in which a carrier supports a palladium-containing compound, and an ammonium salt.

Furthermore, the present inventors have confirmed that, when an olefin, an alcohol, carbon monoxide, and oxygen are reacted with each other in the presence of the above-described catalyst, the compound having the dicarboxylic acid derivative structure in which two carboxy groups and/or carboxylic acid ester groups in total are introduced into a carbon-carbon double bond of the olefin can be produced with a high yield, and has conceived the present invention.

That is, the present invention relates to the following matters.
[1] A method for producing a compound having a dicarboxylic acid derivative structure, the method including:
   a reaction step of reacting an olefin, an alcohol, carbon monoxide, and oxygen with each other in a presence of a heterogeneous catalyst in which a carrier supports a palladium-containing compound, and an ammonium salt.
[2] The method for producing a compound having a dicarboxylic acid derivative structure according to [1],
   in which the ammonium salt is ammonium chloride.
[3] The method for producing a compound having a dicarboxylic acid derivative structure according to [1] or [2],
   in which the olefin is an alicyclic olefin.
[4] The method for producing a compound having a dicarboxylic acid derivative structure according to [1] or [2],
   in which the olefin is a compound represented by Formula (1). 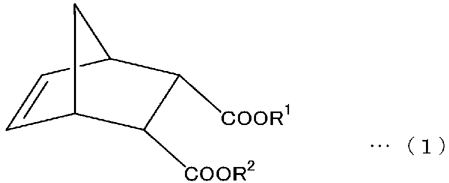 (in Formula (1), R¹ and R² each independently represent an alkyl group having 1 or more and 5 or less carbon atoms)
[5] The method for producing a compound having a dicarboxylic acid derivative structure according to any one of [1] to [4],
   in which the compound having the dicarboxylic acid derivative structure is a compound represented by Formula (2). 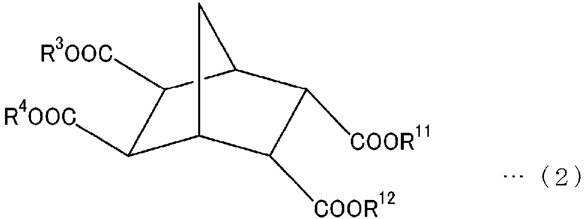 (in Formula (2), R¹¹, R¹², R³, and R⁴ each independently represent a hydrogen atom or an alkyl group having 1 or more and 5 or less carbon atoms)

### Advantageous Effects of Invention

In the method for producing a compound having a dicarboxylic acid derivative structure according to the present invention, an olefin, an alcohol, carbon monoxide, and oxygen are reacted with each other in the presence of a heterogeneous catalyst in which a carrier supports a palladium-containing compound, and an ammonium salt. Therefore, the compound having the dicarboxylic acid derivative structure, in which two carboxy groups and/or carboxylic acid ester groups in total are introduced into a carbon-carbon double bond of the olefin, can be produced with a high yield.

### DESCRIPTION OF EMBODIMENTS

Hereinafter, the method for producing a compound having a dicarboxylic acid derivative structure according to the embodiment of the present invention will be described in detail. The present invention is not limited to embodiments shown below.

The method for producing a compound having a dicarboxylic acid derivative structure according to the present embodiment includes a reaction step of reacting an olefin, an alcohol, carbon monoxide, and oxygen with each other in the presence of a heterogeneous catalyst in which a carrier supports a palladium-containing compound, and an ammonium salt.

In the reaction step of the production method according to the present embodiment, since the heterogeneous catalyst in which a carrier supports a palladium-containing compound and the ammonium salt are used as the catalyst, a reaction rate of a dicarboxylation reaction of the olefin is sufficiently fast. Therefore, it is possible to produce the compound having the dicarboxylic acid derivative structure, in which two carboxy groups (-COOH) and/or carboxylic acid ester groups (-COOR (R is an alkyl group derived from an alcohol (R-OH) used as a raw material)) in total are introduced into a carbon-carbon double bond of the olefin (unsaturated hydrocarbon), which is a target substance, with a high yield.

In addition, in the method for producing a compound having a dicarboxylic acid derivative structure according to the present embodiment, since the ammonium salt is used, there is no possibility that a precipitate derived from a metal is generated by the reaction, for example, as in a case in which a metal salt is used in a conventional production method. Furthermore, since the production method according to the present embodiment is advantageous in terms of cost, the production method is industrially suitable.

### "Olefin"

**In** the method for producing a compound having a dicarboxylic acid derivative structure according to the present embodiment, the olefin used as a raw material can be appropriately determined according to a structure of the compound having the dicarboxylic acid derivative structure, which is a target substance. The olefin used as a raw material may be a chain-like olefin or an alicyclic olefin, and may have a substituent.

Examples of the chain-like olefin include ethylene, propylene, butadiene, pentene, hexene, octene, and dodecene.

Examples of the alicyclic olefin include cyclopentene, norbornene, dicyclopentadiene, cyclooctene, norbornadiene, tricyclopentadiene, ethylidenenorbornene, 3a,4,7,7a-tetrahydroindene, and 4-vinyl-1-cyclohexene. When the alicyclic olefin is used as a raw material, the effect of improving the yield by the production method according to the present embodiment is remarkably obtained, which is preferable.

A substituent which may be contained in the olefin used as a raw material is not particularly limited as long as it is a substituent stable under weakly acidic conditions; and examples thereof include a carboxylic acid ester group having an alkyl group, a carboxy group, a formyl group, a hydroxy group, a tertiary amine, and an acetal. The carboxylic acid ester group having an alkyl group is preferably a carboxylic acid ester group having an alkyl group with 1 or more and 5 or less carbon atoms, and more preferably a carboxylic acid ester group having an alkyl group with 1 or more and 3 or less carbon atoms. This is because, by the production method according to the present embodiment, the compound having the dicarboxylic acid derivative structure, which has a carboxylic acid ester group and is industrially useful, can be produced with a high yield.

The number of substituents which may be contained in the olefin may be one or may be a plurality, and can be appropriately determined depending on the structure of the compound having the dicarboxylic acid derivative structure, which is a target substance.

Specifically, the olefin used as a raw material is preferably norbornenes selected from, for example, bicyclo[2.2.1]hept-2-ene (common name: norbornene), 5-methyl-bicyclo[2.2.1]hept-2-ene, bicyclo[2.2.1]hept-5-ene-2-carboxylic acid, methyl ester, ethyl ester, or propyl ester of bicyclo[2.2.1]hept-5-ene-2-carboxylic acid, 2-methyl-bicyclo[2.2.1]hept-5-en-2-carboxylic acid, methyl ester, ethyl ester, or propyl ester of 2-methyl-bicyclo[2.2.1]hept-5-en-2-carboxylic acid, bicyclo[2.2.1]hept-5-ene-2,3-dicarboxylic acid, and methyl ester, ethyl ester, or propyl ester of bicyclo[2.2.1]hept-5-ene-2,3-dicarboxylic acid.

The olefin used as a raw material is preferably a compound represented by Formula (1). This is because, when the compound represented by Formula (1) is used as a raw material, a tetracarboxylic acid derivative which is industrially useful can be produced with a high yield by the production method according to the present embodiment. 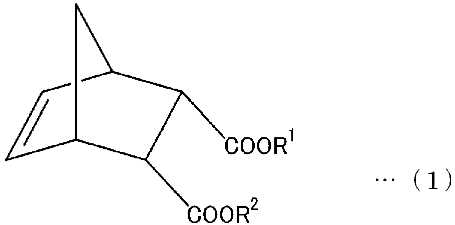 (in Formula (1), R¹ and R² each independently represent an alkyl group having 1 or more and 5 or less carbon atoms)

In the compound represented by Formula (1), R¹ and R² each independently represent an alkyl group having 1 or more and 5 or less carbon atoms, preferably an alkyl group having 1 or more and 3 or less carbon atoms. R¹ and R² may be the same or different from each other. Since the compound having the dicarboxylic acid derivative structure, which is industrially useful, can be produced with a high yield by the production method according to the present embodiment, R¹ and/or R² is particularly preferably a methyl group.

The compound represented by Formula (1) can be easily produced by a known method such as a method adopting a Diels-Alder reaction.

### "Alcohol"

**In** the method for producing a compound having a dicarboxylic acid derivative structure according to the present embodiment, the alcohol used as a raw material can be appropriately determined according to the structure of the compound having the dicarboxylic acid derivative structure, which is a target substance. As the alcohol used as a raw material, an alkyl alcohol (R-OH; R is an alkyl group) is preferable. Specific examples thereof include methanol, ethanol, propanol, isopropanol, butanol, pentanol, hexanol, octanol, and dodecanol. Among the above, as the alcohol, an alcohol in which R is an alkyl group having 1 or more and 5 or less carbon atoms is preferable. This is because the compound having the dicarboxylic acid derivative structure can be produced with a higher yield. Since the compound having the dicarboxylic acid derivative structure, which is industrially useful, can be produced with a high yield by the production method according to the present embodiment, it is particularly preferable to use methanol as the alcohol.

### "Carbon monoxide"

In the method for producing a compound having a dicarboxylic acid derivative structure according to the present embodiment, an amount of the carbon monoxide used as a raw material is preferably an amount such that a partial pressure of the carbon monoxide in the atmosphere in which the dicarboxylation reaction of the olefin is carried out is in a range of 0.01 MPa (absolute pressure; the same applies hereinafter) to 10 MPa, and more preferably an amount such that the partial pressure of the carbon monoxide is in a range of 0.1 MPa to 5.0 MPa. When the partial pressure of the carbon monoxide in the atmosphere in which the dicarboxylation reaction of the olefin is carried out is 0.01 MPa or more, the dicarboxylation reaction of the olefin is likely to proceed, and thus the compound having the dicarboxylic acid derivative structure can be produced with a higher yield. When the partial pressure of the carbon monoxide in the atmosphere in which the dicarboxylation reaction of the olefin is carried out is 10 MPa or less, a reaction container is easily procurable from the viewpoint of ease of pressure design of the reaction container, which is preferable.

### "Oxygen"

In the method for producing a compound having a dicarboxylic acid derivative structure according to the present embodiment, an amount of the oxygen used as a raw material is preferably an amount such that a partial pressure of the oxygen in the atmosphere in which the dicarboxylation reaction of the olefin is carried out is in a range of 0.01 MPa (absolute pressure; the same applies hereinafter) to 2.0 MPa, and more preferably an amount such that the partial pressure of the oxygen is in a range of 0.1 MPa to 1.0 MPa. When the partial pressure of the oxygen in the atmosphere in which the dicarboxylation reaction of the olefin is carried out is 0.01 MPa or more, the dicarboxylation reaction of the olefin is likely to proceed, and thus the compound having the dicarboxylic acid derivative structure can be produced with a higher yield. When the partial pressure of the oxygen in the atmosphere in which the dicarboxylation reaction of the olefin is carried out is 2.0 MPa or less, it is possible to prevent the atmosphere during the dicarboxylation reaction of the olefin from being in an explosion range, which is preferable.

**In** the method for producing a compound having a dicarboxylic acid derivative structure according to the present embodiment, the carbon monoxide and/or oxygen used as a raw material may be used by being mixed with a diluent gas such as nitrogen, argon, helium, and carbon dioxide, which does not inhibit the dicarboxylation reaction of the olefin, as necessary. In this case, it is possible to prevent the atmosphere during the dicarboxylation reaction of the olefin from being in an explosion range, which is preferable.

When the carbon monoxide and/or oxygen is used by being mixed with the diluent gas, the carbon monoxide and/or oxygen may be mixed with the diluent gas and then supplied to a reaction apparatus as a mixed gas, or may be supplied to the reaction apparatus separately from the diluent gas and mixed with the diluent gas in the reaction apparatus. In the production method according to the present embodiment, air may be used as the mixed gas of the oxygen and the diluent gas.

In the method for producing a compound having a dicarboxylic acid derivative structure according to the present embodiment, hydrogen gas may be supplied to the atmosphere during the dicarboxylation reaction of the olefin, as necessary. When the atmosphere during the dicarboxylation reaction of the olefin contains hydrogen gas, the hydrogen gas may be supplied to the reaction apparatus after being mixed with the carbon monoxide and/or oxygen, or may be supplied to the reaction apparatus separately from the carbon monoxide and/or oxygen. In addition, an oxogas (mixed gas of carbon monoxide and hydrogen), which is known as a synthesis gas and is one of basic raw materials in C1 chemistry, may be used as a raw material. In this case, the oxogas can be used as an economically inexpensive carbon monoxide source, compared to a case in which pure carbon monoxide gas is used.

After the dicarboxylation reaction of the olefin, the residual gas containing any one or more of the carbon monoxide, the oxygen, or the diluent gas used as necessary, remaining in the reaction apparatus, may be recovered from the reaction apparatus, the concentration may be adjusted, and the residual gas may be used again in the dicarboxylation reaction of the olefin.

"Heterogeneous catalyst in which carrier supports palladium-containing compound"

The heterogeneous catalyst used in the present embodiment is a catalyst in which a carrier supports a palladium-containing compound. Only one kind of the heterogeneous catalyst may be used, or two or more kinds thereof may be used.

In the method for producing a compound having a dicarboxylic acid derivative structure according to the present embodiment, since the heterogeneous catalyst in which a carrier supports a palladium-containing compound is used as the catalyst, the compound having the dicarboxylic acid derivative structure, which is a target substance, can be produced with a high yield, for example, as compared with a case in which a homogeneous catalyst containing palladium is used.

The reason why a preferred result is obtained by using the heterogeneous catalyst is not well known. **In** the dicarboxylation reaction of the olefin, the palladium-containing compound functions as a catalyst, whereby conversion of the olefin into the compound having the dicarboxylic acid derivative structure is promoted and proceeds. Thereafter, it is considered that the palladium-containing compound, which is changed by the reaction, is returned (is catalyzed) to a state in which the compound can be used again as the catalyst, due to the action of the ammonium salt and the oxygen present in the reaction system. It is presumed that there is some reason for the phenomenon of returning to the state in which the compound can be used again as the catalyst here to be advantageous when the heterogeneous catalyst is used as compared with a case in which the homogeneous catalyst is used.

The heterogeneous catalyst used in the present embodiment can be easily separated from the reaction solution by a method of centrifugation and/or a method of filtration after the dicarboxylation reaction of the olefin. Furthermore, the heterogeneous catalyst separated from the reaction solution can be used again for the dicarboxylation reaction of the olefin, or the like. From these facts, the heterogeneous catalyst is preferable as a catalyst used in an industrial production method, as compared with the homogeneous catalyst.

As the carrier of the heterogeneous catalyst, for example, activated carbon, titania, zirconia, silica, alumina, silica alumina, diatomaceous earth, magnesia, pumice stone, molecular sieve, or the like can be used. Among the above, it is preferable to use activated carbon and/or alumina as the carrier. These carriers are easily procurable, and have a high specific surface area, and thus high activity can be obtained by dispersing fine particles consisting of the palladium-containing compound in the carrier, and resistance to poisoning is also high.

Only one kind of the carrier of the heterogeneous catalyst may be used, or two or more kinds thereof may be used.

A shape and a size of the carrier of the heterogeneous catalyst are not particularly limited, and it is preferable that the carrier has sufficient strength and has a shape in which a sufficient contact area with the raw materials can be secured.

Specifically, as the carrier of the heterogeneous catalyst, for example, a spherical carrier having an average particle diameter of 0.020 mm to 0.36 mm can be used.

In addition, as the carrier of the heterogeneous catalyst, for example, a carrier having a specific surface area of 5 m²/g to 1,500 m²/g can be used. In addition, as the carrier of the heterogeneous catalyst, for example, a carrier having an average pore size of 1 nm to 50 nm can be used. The above-described value is a numerical value obtained by measurement according to a BET method (nitrogen adsorption method).

The palladium-containing compound supported on the carrier of the heterogeneous catalyst may be metallic palladium or a palladium compound. Examples of the palladium compound which can be used as the palladium-containing compound include inorganic acid salts of palladium, such as palladium oxide, palladium nitrate, palladium sulfate, palladium chloride, and palladium phosphate; halogenated palladium such as palladium chloride and palladium bromide; and organic acid salts of palladium, such as palladium acetate, palladium propionate, and palladium benzoate. Among the above, as the palladium-containing compound, it is preferable to use metallic palladium. This is because the dicarboxylation reaction of the olefin is effectively promoted, and thus the compound having the dicarboxylic acid derivative structure, in which two carboxy groups and/or carboxylic acid ester groups in total are introduced into a carbon-carbon double bond of the olefin, can be produced with a high yield.

Only one kind of the palladium-containing compound supported on the carrier of the heterogeneous catalyst may be used, or two or more kinds thereof may be used.

An amount of the palladium-containing compound supported in the heterogeneous catalyst (content of palladium atoms) is not particularly limited, and for example, it can be set to 0.1% by mass to 20% by mass, preferably 0.5% by mass to 10% by mass, and it can be appropriately determined according to the amount of the heterogeneous catalyst used with respect to the amount of the olefin used as a raw material, and the like.

The heterogeneous catalyst in which the carrier supports the palladium-containing compound is not particularly limited. For example, when alumina is used as the carrier, the heterogeneous catalyst can be produced by a method shown below, or the like.

A 250 µL aqueous solution of palladium (II) nitrate having a concentration of 200 g/L is prepared. 0.95 g of an alumina carrier is added to the aqueous solution of palladium (II) nitrate, and the mixture is stirred at normal temperature for 30 minutes. Thereafter, water is removed by vacuum drying. The obtained residue is sintered in air at a temperature of 550°C for 4 hours to obtain an alumina-supported palladium catalyst in which palladium oxide (II) is supported on alumina, which is a heterogeneous catalyst. In this case, the amount of the obtained palladium supported in the heterogeneous catalyst (content of palladium atoms) is 5% by mass.

As the heterogeneous catalyst in which the carrier supports the palladium-containing compound, a commercially available heterogeneous catalyst may be used. Examples of the commercially available heterogeneous catalyst include a heterogeneous catalyst in which activated carbon supports metallic palladium (Scitem; YMC CO., LTD.), barium sulfate-supported Pd (manufactured by TOKYO CHEMICAL INDUSTRY CO., LTD.), calcium carbonate-supported Pd (manufactured by TOKYO CHEMICAL INDUSTRY CO., LTD.), and activated carbon-supported Pd (manufactured by Sigma-Aldrich Co. LLC).

An amount of the palladium-containing compound contained in the heterogeneous catalyst is preferably 1.0 µmol% to 10 mol% and more preferably 5.0 µmol% to 1.0 mol% with respect to 1 mol of the olefin used in the reaction. When the amount of the palladium-containing compound contained in the heterogeneous catalyst is 1.0 µmol% or more with respect to 1 mol of the olefin, the dicarboxylation reaction of the olefin is effectively promoted, and thus the compound having the dicarboxylic acid derivative structure, in which two carboxy groups and/or carboxylic acid ester groups in total are introduced into a carbon-carbon double bond of the olefin, can be produced with a high yield. When the amount of the above-described palladium-containing compound is 10 mol% or less, side reactions in the dicarboxylation reaction of the olefin can be effectively suppressed, and thus the compound having the dicarboxylic acid derivative structure, which is a target substance, can be produced with a higher yield.

The heterogeneous catalyst used in the present embodiment can be separated from the reaction solution by a method of centrifuging the reaction solution and/or a method of filtering the reaction solution, after diluting the reaction solution after the dicarboxylation reaction of the olefin as necessary. The heterogeneous catalyst separated from the reaction solution can be used again in the dicarboxylation reaction of the olefin.

### "Ammonium salt"

Examples of the ammonium salt used in the present embodiment include ammonium chloride, ammonium bromide, ammonium iodide, methylammonium chloride, benzyltrimethylammonium chloride, and a tetraalkylammonium salt. Examples of the tetraalkylammonium salt include tetrabutylammonium chloride. Among the above, as the ammonium salt, a halogen-containing ammonium salt is preferably used, and ammonium chloride is particularly preferably used. The halogen-containing ammonium salt effectively contributes to regeneration and catalysis of the palladium-containing compound, which is changed due to the reaction, by reoxidation, and stabilizes the palladium-containing compound contained in the heterogeneous catalyst by the halide ion of the counter anion. This is because the dicarboxylation reaction of the olefin is effectively promoted, and thus the compound having the dicarboxylic acid derivative structure, in which two carboxy groups and/or carboxylic acid ester groups in total are introduced into a carbon-carbon double bond of the olefin, can be produced with a high yield.

Only one kind of the ammonium salt used in the present embodiment may be used, or two or more kinds thereof may be used.

An amount of the ammonium salt used is preferably 1.0 molar amount to 10 molar amount, and more preferably 2.0 molar amount to 5.0 molar amount with respect to 1 mol of the olefin used as a raw material. When the used amount of the ammonium salt contained in the heterogeneous catalyst is 1.0 molar amount or more with respect to 1 mol of the olefin, the dicarboxylation reaction of the olefin is effectively promoted, and thus the compound having the dicarboxylic acid derivative structure, in which two carboxy groups and/or carboxylic acid ester groups in total are introduced into a carbon-carbon double bond of the olefin, can be produced with a high yield. When the above-described used amount of the ammonium salt is 10 molar amount or less, precipitation of the ammonium salt in the dicarboxylation reaction of the olefin can be effectively suppressed, and thus the compound having the dicarboxylic acid derivative structure, which is a target substance, can be produced with a higher yield.

### "Solvent"

**In** the method for producing a compound having a dicarboxylic acid derivative structure according to the present embodiment, the reaction solution for the dicarboxylation reaction of the olefin may contain one or two or more kinds of solvents which do not inhibit the dicarboxylation reaction of the olefin, as necessary. In the method for producing a compound having a dicarboxylic acid derivative structure according to the present embodiment, the alcohol used as a raw material may be substantially used as the solvent.

Examples of the solvent which can be used in the production method according to the present embodiment include ethers such as diethyl ether, dipropyl ether, methyl ethyl ether, tetrahydrofuran, dioxane, ethylene glycol diethyl ether, and triethylene glycol dimethyl ether; ketones such as acetone, methyl ethyl ketone, and acetophenone; esters such as methyl acetate, ethyl acetate, and methyl propionate; aromatic hydrocarbons such as benzene, toluene, p-xylene, ethylbenzene, chlorobenzene, and dichlorobenzene, or substituted compounds thereof; aliphatic or alicyclic hydrocarbons such as n-hexane, n-pentane, isooctane, and cyclohexane; carbonates such as propylene carbonate and dimethyl carbonate; nitriles such as acetonitrile and benzonitrile; amide compounds such as dimethylformamide; and sulfone compounds such as sulfolane.

**In** addition, when the solvent does not dissolve the ammonium salt to be used, a small amount of distilled water may be added to assist the dissolution of the ammonium salt.

### "Reaction conditions"

The reaction step in the method for producing a compound having a dicarboxylic acid derivative structure according to the present embodiment is preferably a step shown below. First, a predetermined amount of the olefin, the alcohol, the above-described heterogeneous catalyst, the ammonium salt, and the solvent used as necessary are put into a reaction container of a reaction apparatus. Next, a dicarboxylation reaction of the olefin is carried out by supplying the carbon monoxide, the oxygen, and the diluent gas used as necessary into the reaction container of the reaction apparatus, and stirring the inside of the reaction container by a known method.

The total amount of the carbon monoxide required for the dicarboxylation reaction of the olefin may be supplied to the reaction apparatus in a lump, or may be continuously or intermittently supplied thereto. In the present embodiment, it is particularly preferable to carry out the reaction while supplementing the carbon monoxide consumed by the dicarboxylation reaction of the olefin, so that the partial pressure of the carbon monoxide in the atmosphere in which the dicarboxylation reaction of the olefin is carried out is maintained in the range of 0.01 MPa to 10 MPa.

The total amount of the oxygen required for the dicarboxylation reaction of the olefin may be supplied to the reaction apparatus in a lump, or may be continuously or intermittently supplied thereto. In the present embodiment, it is particularly preferable to carry out the reaction while supplementing the oxygen consumed by the dicarboxylation reaction of the olefin, so that the partial pressure of the oxygen in the atmosphere in which the dicarboxylation reaction of the olefin is carried out is maintained in the range of 0.01 MPa to 2.0 MPa.

The reaction conditions of the reaction step in the method for producing a compound having a dicarboxylic acid derivative structure according to the present embodiment can be appropriately determined depending on the kind of the olefin and alcohol as the raw material, the kind of the above-described heterogeneous catalyst and ammonium salt as the catalyst, and the like, within a range in which the dicarboxylation reaction of the olefin proceeds.

Regarding the reaction conditions, it is preferable to satisfy any one or more of the following conditions of (a) reaction apparatus, (b) reaction pressure, (c) reaction temperature, and (d) reaction time.

### (a) Reaction apparatus

**In** the present embodiment, it is preferable that the reaction apparatus used for the dicarboxylation reaction of the olefin includes a pressure-resistant reaction container which can enclose the olefin, the alcohol, the carbon monoxide, and the oxygen together with the above-described heterogeneous catalyst and ammonium salt, and can be sealed under pressure. Specifically, as the reaction apparatus, for example, it is preferable to use an autoclave including a pressure-resistant reaction container made of stainless steel such as SUS304, SUS316, and SUS316L, titanium, tantalum, nickel, HASTELLOY, Inconel, and the like.

### (b) Reaction pressure

As a pressure in the atmosphere in which the dicarboxylation reaction of the olefin is carried out (pressure in the pressure-resistant reaction container), it is preferable that the partial pressures of the carbon monoxide and the oxygen are each within the above-described range. Furthermore, the pressure (total pressure) in the pressure-resistant reaction container is preferably in a range of 0.1 MPa (absolute pressure; the same applies hereinafter) to 10 MPa, and more preferably in a range of 0.1 MPa to 5 MPa. When the pressure in the pressure-resistant reaction container is 0.1 MPa or more, the dicarboxylation reaction of the olefin is effectively promoted, and thus the dicarboxylic acid derivative can be produced with a higher yield. In addition, when the pressure in the pressure-resistant reaction container is 10 MPa or less, specification of the reaction container may be a container which can be used for general industrial applications in terms of process design, and thus a special structure is not necessary, which is preferable.

### (c) Reaction temperature

A temperature of the atmosphere in which the dicarboxylation reaction of the olefin is carried out (temperature in the pressure-resistant reaction container) is preferably in a range of 0°C to 150°C and more preferably 20°C to 100°C. When the temperature in the pressure-resistant reaction container is 0°C or higher, the dicarboxylation reaction of the olefin is effectively promoted, and thus the compound having the dicarboxylic acid derivative structure can be produced with a higher yield. When the temperature in the pressure-resistant reaction container is 150°C or lower, side reactions in the dicarboxylation reaction of the olefin can be effectively suppressed, and thus the compound having the dicarboxylic acid derivative structure can be produced with a higher yield.

### (d) Reaction time

A reaction time in the reaction step is preferably in a range of 0.5 hours to 100 hours, and more preferably 3 to 48 hours. When the reaction time is 0.5 hours or longer, the dicarboxylation reaction of the olefin sufficiently proceeds, and thus the compound having the dicarboxylic acid derivative structure can be produced with a higher yield. When the reaction time is 100 hours or shorter, the compound having the dicarboxylic acid derivative structure can be efficiently produced, and moreover, side reactions in the dicarboxylation reaction of the olefin can be effectively suppressed.

### "Compound having dicarboxylic acid derivative structure"

In the method for producing a compound having a dicarboxylic acid derivative structure according to the present embodiment, the compound having the dicarboxylic acid derivative structure, in which two carboxy groups (-COOH) and/or carboxylic acid ester groups (-COOR (R is an alkyl group derived from the alcohol used as a raw material)) in total are introduced into a carbon-carbon double bond of the olefin (unsaturated hydrocarbon), which is a target substance, is obtained with a high yield. The obtained compound having a dicarboxylic acid derivative structure is a compound in which the carboxy group or the carboxylic acid ester group is bonded to each of adjacent carbon atoms, and is useful as a raw material for various compounds, an additive to a resin, and the like. Specifically, for example, a compound having an imide bond, which is industrially useful, can be produced by reacting the compound having the dicarboxylic acid derivative structure, which is produced by production method according to the present embodiment, with an amino compound. Therefore, the compound having the dicarboxylic acid derivative structure, which is produced by the production method according to the present embodiment, can be used as a raw material of a compound having an imide bond.

In the production method according to the present embodiment, when an alicyclic olefin is used as a raw material, a compound having a cyclic skeleton and having a carboxy group or a carboxylic acid ester group bonded to each of adjacent carbon atoms is obtained as the compound having the dicarboxylic acid derivative structure. The obtained compound having a dicarboxylic acid derivative structure can be used as a raw material for a compound having an imide bond, which is more useful in industry. Therefore, the effect of improving the yield by the production method according to the present embodiment is remarkable.

For example, in the production method according to the present embodiment, when the compound represented by Formula (1), which is an alicyclic olefin, is used as a raw material, a compound represented by Formula (2) is obtained as the compound having the dicarboxylic acid derivative structure. In this case, R¹¹ and R¹² in Formula (2) are each a group derived from R¹ and R² in Formula (1). 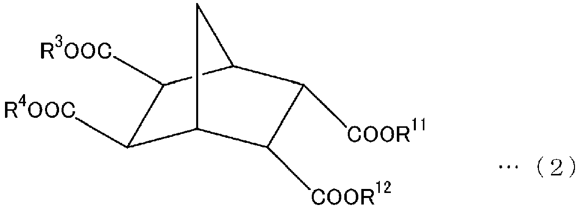 (in Formula (2), R¹¹, R¹², R³, and R⁴ each independently represent a hydrogen atom or an alkyl group having 1 or more and 5 or less carbon atoms)

In the compound represented by Formula (2), R¹¹, R¹², R³, and R⁴ each independently represent a hydrogen atom or an alkyl group having 1 or more and 5 or less carbon atoms; and a hydrogen atom or an alkyl group having 1 or more and 3 or less carbon atoms is preferable. R¹¹, R¹², R³, and R⁴ may be different from each other, or may be the same in part or in whole.

In the compound represented by Formula (2), R¹¹ and R¹² are groups derived from R¹ and R² in the compound represented by Formula (1) used as a raw material.

R¹¹ in Formula (2) may be the same as R¹ in Formula (1), or may be a hydrogen atom due to hydrolysis of -COOR¹ which is an ester moiety in Formula (1) to form a carboxy group (-COOH). In addition, R¹² in Formula (2) may be the same as R² in Formula (1), or may be a hydrogen atom due to hydrolysis of -COOR² in Formula (1) to form a carboxy group.

R³ and R⁴ are groups derived from the alcohol used as a raw material. For example, when an alkyl alcohol is used as the alcohol, R³ and R⁴ are groups derived from an alkyl group of the alkyl alcohol. When only one kind of the alcohol is used as a raw material, R³ and R⁴ are likely to be the same group. In consideration of ease of separation and purification of the target substance from the reaction solution after the reaction, it is preferable that R³ and R⁴ are the same. Since the target substance is the compound having the dicarboxylic acid derivative structure which is industrially useful, it is particularly preferable that R³ and/or R⁴ is a methyl group.

A part or all of R¹¹, R¹², R³, and R⁴ may be a hydrogen atom. That is, the compound represented by Formula (2) may be a carboxylic acid. The compound in which a part or all of R¹¹, R¹², R³, and R⁴ are hydrogen atoms is generated by one or more reactions of the following <1> to <3> as the reaction for generating the dicarboxylic acid derivative structure.
<1> A water molecule present in the reaction system during the reaction of the carbon-carbon double bond in the compound represented by Formula (1) reacts instead of the alcohol used as a raw material.
<2> One or both of -COOR³ and -COOR⁴, which are ester moieties introduced into the carbon-carbon double bond in the compound represented by Formula (2), are hydrolyzed by reacting with a water molecule present in the reaction system.
<3> One or both of -COOR¹ and -COOR², which are ester moieties in the compound represented by Formula (1), are hydrolyzed by reacting with a water molecule present in the reaction system.

**In** the production method according to the present embodiment, when evaluating the yield of the reaction, the yield of the compound in which R¹¹, R¹², R³, and R⁴ are each an alkyl group having 1 or more and 5 or less carbon atoms, the yield of the compound in which a part or all of R¹¹, R¹², R³, and R⁴ are hydrogen atoms, and the total yield thereof can be evaluated.

The compound represented by Formula (2) can be used, for example, as a raw material of a diimide compound. Specifically, the ester group of the compound represented by Formula (2) is hydrolyzed to form a carboxylic acid structure. As a result, a tetracarboxylic acid compound in which all of R¹¹, R¹², R³, and R⁴ in the compound represented by Formula (2) are substituted with a hydrogen atom is obtained. Thereafter, an imide compound is produced by a known method using the obtained tetracarboxylic acid compound.

When the compound represented by Formula (2) is used as the raw material of the diimide compound, the compound is used after the ester group is hydrolyzed to form a carboxylic acid structure. Therefore, in the production method according to the present embodiment, when the compound in which R¹¹, R¹², R³, and R⁴ are each an alkyl group having 1 or more and 5 or less carbon atoms is obtained as the compound represented by Formula (2), when the compound in which a part or all of R¹¹, R¹², R³, and R⁴ are hydrogen atoms is obtained as the compound represented by Formula (2), or when a mixture of these compounds is generated, the mixture can be similarly used as the raw material in the synthesis of the diimide compound.

The compound represented by Formula (2) has a structure in which four groups of -COOR¹¹, -COOR¹², -COOR³, and -COOR⁴ are bonded to a norbornene skeleton. Therefore, the compound represented by Formula (2) can be used as a raw material for a polyimide having an industrially useful norbornene skeleton and imide bond, by reaction with an amino compound. Accordingly, the effect of improving the yield by the production method according to the present embodiment is remarkable.

### Examples

Hereinafter, the present invention will be described in more detail with reference to Examples and Comparative Examples. The present invention is not limited to Examples described below.

### (Reference Example)

### [Synthesis of compound represented by Formula (1)]

10.3 g (57 mmol) of bicyclo[2.2.1]hept-5-ene-2endo,3endo-dicarboxylic acid (may be described as "bicyclo[2.2.1]hept-5-ene-2,3-dicarboxylic acid") was dissolved in 200 mL of N,N-dimethylformamide (manufactured by FUJIFILM Wako Pure Chemical Corporation). 31.3 g of potassium carbonate (FUJIFILM Wako Pure Chemical Corporation, 226 mmol) and 14 mL of iodomethane (manufactured by TOKYO CHEMICAL INDUSTRY CO., LTD., 226 mmol) were added to the solution, and the mixture was stirred and reacted at room temperature for 48 hours.

Water and ethyl acetate were added to the reaction solution after the reaction, and the reaction solution was subjected to liquid separation. The organic layer was washed with water and then dried over anhydrous sodium sulfate, the solvent was distilled off under reduced pressure, and the residue was purified by column chromatography to obtain, as a white solid, dimethyl ester of bicyclo[2.2.1]hept-5-ene-2endo,3endo-dicarboxylic acid which is a reaction product (yield amount: 10.0 g, yield: 84%).

The obtained white solid was analyzed under the following analysis conditions using gas chromatography shown below. As a result, it was confirmed that the obtained white solid was the dimethyl ester of bicyclo[2.2.1]hept-5-ene-2endo,3endo-dicarboxylic acid as the compound represented by Formula (1) (R¹ and R² in Formula (1) were methyl groups).

### (Analysis conditions of gas chromatography)

As the gas chromatography, GC6850 Series II manufactured by Agilent Technologies, Inc. was used. A flame ionization detector (FID) was used as a detector. A J&W HP-1 column (thickness: 0.25 µm, 0.32 mm I.D., 30 m) was used as a column. The measurement was performed at a column temperature of 40°C and a measurement temperature of 40°C to 240°C. Specifically, the sample was held at 40°C for 5 minutes, heated to 240°C at a heating rate of 10°C per minute, and held at 240°C for 5 minutes according to a program.

### (Example 1)

### [Production of compound having dicarboxylic acid derivative structure]

200 mg (0.95 mmol) of the dimethyl ester of bicyclo[2.2.1]hept-5-ene-2endo,3endo-dicarboxylic acid as the compound represented by Formula (1) (R¹ and R² in Formula (1) were methyl groups) synthesized by the above-described method, 10.2 mg (4.8 µmol) of activated carbon-supported palladium (manufactured by YMC CO., LTD.), and 127 mg (2.4 mmol) of ammonium chloride (manufactured by Sigma-Aldrich Co. LLC) were weighed and put into a cylindrical container made of glass.

As the activated carbon-supported palladium (manufactured by YMC CO., LTD.), a carrier containing spherical activated carbon having an average particle diameter of 0.36 mm, a specific surface area of 1,371 m²/g, and an average pore size of 1.7 nm, to which metallic palladium was supported, was used, in which the supported amount of palladium (content of palladium atoms) was 5% by mass. The specific surface area of the activated carbon is a numerical value obtained by measurement by a BET method (surface area measurement method).

3.0 mL of methanol (manufactured by FUJIFILM Wako Pure Chemical Corporation) and 1.0 mL of distilled water (added to dissolve ammonium chloride) were further added to the above-described cylindrical container. Thereafter, the cylindrical container containing the above-described materials was placed in a pressure-resistant reaction container of an autoclave, and sealed.

Oxygen was supplied to the pressure-resistant reaction container with a partial pressure of the oxygen of 1.0 MPa, carbon monoxide was supplied thereto with a partial pressure of the carbon monoxide of 3.5 MPa, and the total pressure in the pressure-resistant reaction container was set to 4.5 MPa. Thereafter, the mixture was reacted at 60°C for 24 hours while vigorously stirring the inside of the pressure-resistant reaction container.

After completion of the reaction, the pressure was released by cooling the pressure-resistant reaction container the temperature in the pressure-resistant reaction container reached room temperature, and the reaction solution in the cylindrical container was collected. A part of the reaction solution was sampled as a test specimen by the method shown below, and analyzed under the above-described analysis conditions using the above-described gas chromatography to identify the reaction product. As a result, it was confirmed that the reaction product was tetramethyl ester of bicyclo[2.2.1]heptane-2endo,3endo,5exo,6exo-tetracarboxylic acid (the compound represented by Formula (2) (R¹¹, R¹², R³, and R⁴ in Formula (2) were methyl groups)), which was a target substance of Example 1.

### (Sampling method for test specimen)

50 µL of diethylene glycol dimethyl ether as an internal standard substance was added to the reaction solution in the cylindrical container after the reaction, and transferred together with the reaction solution to a test tube with a screw neck, and the mixture was further diluted by adding 7 mL of methanol. The diluted reaction solution was centrifuged at room temperature and a rotation speed of 3,000 rpm for 5 minutes to precipitate the heterogeneous catalyst contained in the diluted reaction solution, and the supernatant solution was collected (sampled) as a test specimen for analysis.

**In** addition, using the identification result of the reaction product, a conversion rate of the compound represented by Formula (1), the yield of the target substance (compound in which R¹¹, R¹², R³, and R⁴ in Formula (2) were methyl groups), the yield of the hydrolyzate of the target substance of Example 1 (total of compounds in which some or all of R¹¹, R¹², R³, and R⁴ in Formula (2) were hydrogen atoms), and the total yield thereof (yield of target substance + yield of hydrolyzate) were calculated. The results are shown in Table 1.

As shown in Table 1, in Example 1, the conversion rate of the reaction was 100%. In addition, the yield of the tetramethyl ester of bicyclo[2.2.1]heptane-2endo,3endo,5exo,6exo-tetracarboxylic acid (compound represented by Formula (2) (R¹¹, R¹², R³, and R⁴ in Formula (2) were methyl groups)) as the target substance was 100%.

**[Table 1]**

| | Heterogeneous catalyst | Note | Conversion rate (%) | Yield of target substance (%) | Yield of hydrolysate (%) | Total yield (%) |
|---|---|---|---|---|---|---|
| Example 1 | Activated carbon-supported palladium | | 100 | 100 | 0 | 100 |
| Example 2 | Alumina-supported palladium | | 98 | 90 | 7 | 97 |
| Comparative Example 1 | Activated carbon-supported palladium | Not adding oxygen | 0 | 0 | 0 | 0 |
| Comparative Example 2 | - | Palladium chloride (homogeneous catalyst) | 100 | 16 | 6 | 22 |

### (Example 2)

A compound having a dicarboxylic acid derivative structure was produced in the same manner as in Example 1, except that alumina-supported palladium in which a carrier containing alumina supported a palladium-containing compound, which was produced by the method described below, was used as the heterogeneous catalyst.

The obtained reaction product was identified in the same manner as in Example 1. As a result, it was confirmed that the reaction product was a mixture containing the compound in which R¹¹, R¹², R³, and R⁴ in Formula (2) were methyl groups, which was a target substance of Example 2, and the compound in which a part or all of R¹¹, R¹², R³, and R⁴ in Formula (2) were hydrogen atoms, which was a hydrolyzate of the target substance of Example 2.

In addition, using the identification result of the reaction product, same as in Example 1, the conversion rate of the compound represented by Formula (1), the yield of the target substance, the yield of the hydrolyzate, and the total yield thereof (yield of target substance + yield of hydrolyzate) were calculated. The results are shown in Table 1.

As shown in Table 1, in Example 2, the conversion rate of the reaction was 98%, which was a very high result. In addition, the total yield was 97%, which was sufficiently high.

### (Method for producing heterogeneous catalyst (alumina-supported palladium))

The alumina-supported palladium catalyst was prepared by the following procedure.

First, a 250 µL aqueous solution of palladium (II) nitrate having a concentration of 200 g/L was prepared. The palladium concentration in the aqueous solution of palladium (II) nitrate was adjusted such that the supported amount of palladium (content of palladium atoms) in the heterogeneous catalyst produced using the aqueous solution of palladium (II) nitrate was 5% by mass.

0.95 g of an alumina carrier was added to the obtained aqueous solution of palladium (II) nitrate, and the mixture was stirred at room temperature for 30 minutes to impregnate the alumina carrier with the aqueous solution of palladium (II) nitrate. As the alumina carrier, a spherical carrier (JRC-ALO-10; manufactured by Nippon Light Metal Company, Ltd.) having an average particle diameter of 0.0201 mm, a specific surface area of 104 m²/g, and an average pore size of 20.1 nm was used. Thereafter, water was removed from the alumina carrier impregnated with the aqueous solution of palladium (II) nitrate by vacuum drying. The obtained residue was sintered in air at a temperature of 550°C for 4 hours to obtain the alumina-supported palladium catalyst in which palladium oxide (II) was supported on alumina.

### (Comparative Example 1)

A compound having a dicarboxylic acid derivative structure was produced in the same manner as in Example 1, except that the oxygen was not supplied to the pressure-resistant reaction container. However, as shown in Table 1, the conversion rate of the compound represented by Formula (1) was 0%, and the compound represented by Formula (2), which was a target substance, was not produced.

### (Comparative Example 2)

A compound having a dicarboxylic acid derivative structure was produced in the same manner as in Example 1, except that 8.5 mg (5 mol%) of palladium chloride (manufactured by FUJIFILM Wako Pure Chemical Corporation), which was a homogeneous catalyst, was used instead of the heterogeneous catalyst.

The reaction solution after the completion of the reaction was sampled as it was without centrifugation, and analyzed by gas chromatography in the same manner as in Example 1 to identify the reaction product. As a result, it was confirmed that the reaction product was a mixture containing the compound in which R¹¹, R¹², R³, and R⁴ in Formula (2) were methyl groups, which was a target substance of Comparative Example 2, and the compound in which a part or all of R¹¹, R¹², R³, and R⁴ in Formula (2) were hydrogen atoms, which was a hydrolyzate of the target substance of Comparative Example 2.

In addition, using the identification result of the reaction product, same as in Example 1, the conversion rate of the compound represented by Formula (1), the yield of the target substance, the yield of the hydrolyzate, and the total yield thereof (yield of target substance + yield of hydrolyzate) were calculated. The results are shown in Table 1.

As shown in Table 1, in Comparative Example 2, the conversion rate of the reaction was 100%. However, the total yield of the yield of the target substance and the yield of the hydrolyzate was 22%, which was lower than that in Example 1 and Example 2.

### (Examples 3 to 5 and Comparative Examples 3 and 4)

A compound having a dicarboxylic acid derivative structure was produced in the same manner as in Example 1, except that 2.4 mmol of the ammonium salt shown in Table 2 or the nitrogen-containing compound which was not the ammonium salt was used instead of the ammonium chloride.

### (Comparative Example 5)

A compound having a dicarboxylic acid derivative structure was produced in the same manner as in Example 1, except that the ammonium chloride was not added.

The reaction products obtained in Examples 3 to 5 and Comparative Examples 3 to 5 were identified in the same manner as in Example 1. As a result, it was confirmed that the reaction product contained tetramethyl ester of bicyclo[2.2.1]heptane-2endo,3endo,5exo,6exo-tetracarboxylic acid (the compound represented by Formula (2) (R¹¹, R¹², R³, and R⁴ in Formula (2) were methyl groups)), which was a target substance.

In addition, using the identification results of the reaction products obtained in Examples 3 to 5 and Comparative Examples 3 to 5, same as in Example 1, the conversion rate of the compound represented by Formula (1), the yield of the target substance, the yield of the hydrolyzate of the target substance, and the total yield thereof (yield of target substance + yield of hydrolyzate) were calculated. The results are shown in Table 2. Furthermore, Table 2 also shows the amount (mass) of the ammonia salt used, the purchase source, and the results of Example 1.

**[Table 2]**

| | Ammonium salt or nitrogen-containing compound which is not ammonium salt | Used amount (mg) | Purchase source | Conversion rate (%) | Yield of target substance (%) | Yield of hydrolysate (%) | Total yield (%) |
|---|---|---|---|---|---|---|---|
| Example 1 | Ammonium chloride | 127 | Sigma-Aldrich Co. LLC | 100 | 100 | 0 | 100 |
| Example 3 | Methylammonium chloride | 162 | FUJIFILM Wako Pure Chemical Corporation | 100 | 89 | 3 | 92 |
| Example 4 | Benzyltrimethylammonium chloride | 547 | Sigma-Aldrich Co. LLC | 97 | 64 | 4 | 68 |
| Example 5 | Ammonium iodide | 359 | NACALAI TESQUE, INC. | 100 | 64 | 0 | 64 |
| Comparative Example 3 | 1,3-Bis(2,4,6-trimethylphenyl)imidazolinium chloride | 823 | TOKYO CHEMICAL INDUSTRY CO., LTD. | 19 | 8 | 0 | 8 |
| Comparative Example 4 | Pyridine hydrochloride | 277 | TOKYO CHEMICAL INDUSTRY CO., LTD. | 97 | 11 | 13 | 24 |
| Comparative Example 5 | - | - | - | 1 | 1 | 0 | 1 |

As shown in Table 2, in Examples 3 to 5 in which other ammonium salts were used instead of the ammonium chloride, the total yield of the yield of the target substance and the yield of the hydrolyzate was 60% or more, which was sufficiently high.

On the other hand, in Comparative Example 3 and Comparative Example 4 in which the nitrogen-containing compound which was not an ammonium salt was used instead of the ammonium chloride, and in Comparative Example 5 in which the ammonium salt was not added, the yield of the target substance and the total yield were low.

### INDUSTRIAL APPLICABILITY

An olefin, an alcohol, carbon monoxide, and oxygen are reacted with each other in the presence of a heterogeneous catalyst in which a carrier supports a palladium-containing compound, and an ammonium salt. A compound having a dicarboxylic acid derivative structure, in which two carboxy groups and/or carboxylic acid ester groups in total are introduced into a carbon-carbon double bond of the olefin, can be produced with a high yield.

## Claims

1. A method for producing a compound having a dicarboxylic acid derivative structure, the method comprising:
a reaction step of reacting an olefin, an alcohol, carbon monoxide, and oxygen with each other in a presence of a heterogeneous catalyst in which a carrier supports a palladium-containing compound, and an ammonium salt.

2. The method for producing a compound having a dicarboxylic acid derivative structure according to Claim 1,
wherein the ammonium salt is ammonium chloride.

3. The method for producing a compound having a dicarboxylic acid derivative structure according to Claim 1 or 2,
wherein the olefin is an alicyclic olefin.

4. The method for producing a compound having a dicarboxylic acid derivative structure according to Claim 1 or 2,
wherein the olefin is a compound represented by Formula (1), 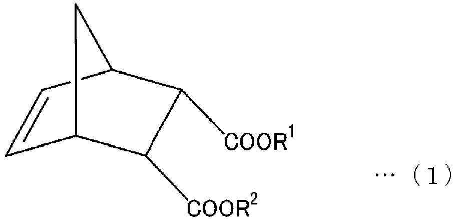 (in Formula (1), R¹ and R² each independently represent an alkyl group having 1 or more and 5 or less carbon atoms).

5. The method for producing a compound having a dicarboxylic acid derivative structure according to Claim 1 or 2,
wherein the compound having a dicarboxylic acid derivative structure is a compound represented by Formula (2), 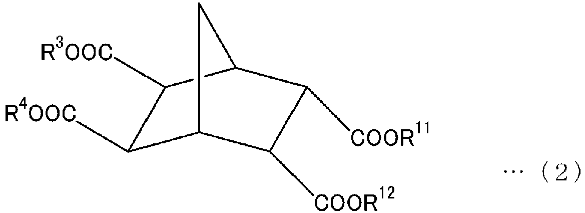 (in Formula (2), R¹¹, R¹², R³, and R⁴ each independently represent a hydrogen atom or an alkyl group having 1 or more and 5 or less carbon atoms).
